(19) Europäisches Patentamt
European Patent Office
Office européen des brevets

(11) Publication number: **0 375 557 A1**

(12) **EUROPEAN PATENT APPLICATION**

(21) Application number: **89403605.2**

(51) Int. Cl.[5]: **C07C 311/19, C07C 311/16, A61K 31/195**

(22) Date of filing: **21.12.89**

(30) Priority: **22.12.88 JP 323885/88**

(43) Date of publication of application: **27.06.90 Bulletin 90/26**

(84) Designated Contracting States: **AT BE CH DE ES FR GB GR IT LI LU NL SE**

(71) Applicant: **TANABE SEIYAKU CO., LTD.**
**2-10, Dosho-machi 3-chome**
**Chuo-ku Osaka(JP)**

(72) Inventor: **Iwakuma, Takeo**
**1-5-7, Fujimi**
**Ageo-shi Saitama-ken(JP)**
Inventor: **Sekine, Yasuo**
**6-22-28 Asahi**
**Kawaguchi-shi Saitama-ken(JP)**
Inventor: **Sasaki, Yasuhiko**
**410 Urawa New heights No 85-1, Jinde**
**Urawa-shi Saitama-ken(JP)**
Inventor: **Ikezawa, Katsuo**
**2-28-28, Kizaki**
**Urawa-shi Saitama-ken(JP)**
Inventor: **Odawara, Akio**
**47-19-301, Akabane-nishi-4-chome**
**Kita-ku Tokyo-to(JP)**

(74) Representative: **Koch, Gustave et al**
**Cabinet PLASSERAUD 84, rue d'Amsterdam**
**F-75009 Paris(FR)**

# (54) Glycine derivative process for preparation thereof, and pharmaceutical composition containing it.

(57) Novel glycine derivative having the formula (I):

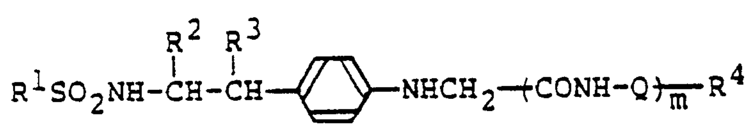

(I)

wherein $R^1$ is a substituted or unsubstituted phenyl group; either $R^2$ or $R^3$ is a lower alkyl group and the other is hydrogen atom, $R^4$ is a protected or nonprotected carboxyl group, Q is a lower alkylene group and m is O or 1
or a pharmaceutically acceptable salt thereof are disclosed. Said derivative (I) and a pharmaceutically acceptable salt thereof have a potent platelet aggregation-inhibiting acitivity.

EP 0 375 557 A1

# GLYCINE DERIVATIVE PROCESS FOR PREPARATION THEREOF, AND PHARMACEUTICAL COMPOSITION CONTAINING IT

The present invention relates to novel glycine derivatives, to processes for preparation thereof,. to pharmaceutical compositions containing them and more particularly to a glycine derivative having a potent platelet aggregation-inhibiting activity.

It has been known that N-[4-(2-benzenesulfonylaminoethyl)phenyl]glycine shows platelet aggregation-inhibiting activity (DE-A-3622865).

For the purpose of developing a novel superior platelet aggregation-inhibiting activity, the present inventors have synthesized a novel glycine derivative having the formula (I), and as a result, found that the novel glycine derivative having the formula (I) exhibits a potent platelet aggregation-inhibiting activity.

In accordance with the present invention, there is proveded a glycine derivative having the formula (I):

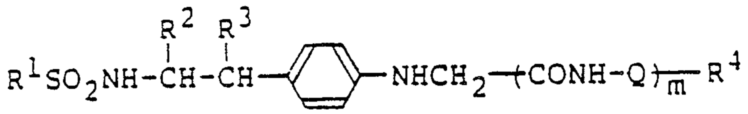

(I)

wherein $R^1$ is a substituted or unsubstituted phenyl group, either $R^2$ or $R^3$ is a lower alkyl group and the other is hydrogen atom, $R^4$ is a protected or nonprotected carboxyl group, Q is a lower alkylene group and m is O or 1;
or a salt thereof.

Also, in accordance with the present invention, there is provided a preparation of the glycine derivative (I).

An object of the present invention is to provide glycine derivatives which show strong platelet aggregation-inhibiting activity as compared with the above known compound.

A further object of the invention is to provide a process for preparing the glycine derivative.

These and other objects of the invention will become apparent from the description hereinafter.

The glycine derivative of the present invention is a glycine derivative having the formula (I).

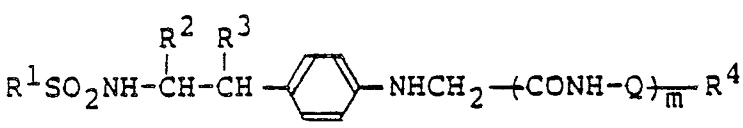

(I)

wherein $R^1$ is a substituted or unsubstituted phenyl group; $R^2$ and $R^3$ are different from each other and either $R^2$ or $R^3$ is a lower alkyl group with 1 to 6, preferably 1 to 3 carbon atoms, more preferably methyl group and the other is hydrogen atom; $R^4$ is free carboxyl group or a protected carboxyl group; Q is a lower alkylene group with 1 to 6, preferably from 1 to 3, more preferably from 2 to 3, carbon atoms; and m is 0 or 1.

Examples of the substituents are, for instance, a halogen atom such as chlorine, a lower alkyl group such as methyl, a lower alkoxy group such as methoxy, nitro group, and the like.

Any protecting group which can be easily removed by a usual manner can be used as the protecting group of the carboxyl group.

Examples of the protecting groups of carboxyl group are, for instance, a lower alkyl group with 1 to 4 carbon atoms, a phenyl-($C_{1-3}$ alkyl) group, a $C_{1-4}$ alkoxy phenyl-($C_{1-3}$ alkyl) group, a nitro phenyl-($C_{1-3}$ alkyl) group, a benzhydryl group, and the like.

The glycine derivative having the formula (I) of the present invention and the salt thereof show excellent thromboxan $A_2$ (hereinafter referred to as "$TxA_2$") antagonistic activities, and are suitable for use as platelet aggregation-inhibitors, the therapeutic treatment, amelioration and/or prophylaxis of a variety of thromboses or embolisms, such as cerebral thrombosis, coronary artery thrombosis, pulmonary thrombosis, pulmonary embolism, peripheral vascular embolism and thromboangiitis, and asthma.

Examples of the glycine derivative of the present invention are those having the formula (I) wherein $R^1$ is phenyl group or a halogenophenyl group, and $R^4$ is free carboxyl group or a protected carboxyl group with, for instance, a lower alkyl group, a phenyl-lower alkyl group, a lower alkoxyphenyl-lower alkyl group, a

nitrophenyl-lower alkyl group and benzhydryl group.

Among them, therapeutically preferred examples of the glycine derivative of the present invention are those having the formula (I) wherein $R^4$ is free carboxyl group or a lower alkoxycarbonyl group.

More preferred examples of the glycine derivataive are those having the formula (I) wherein $R^1$ is a halogenophenyl group, either $R^2$ or $R^3$ is an alkyl group having 1 to 3 carbon atoms and the other is hydrogen atom, $R^4$ is free carboxyl group or an alkoxycarbonyl group having 2 to 4 carbon atoms and Q is an alkylene group having 1 to 3 carbon atoms.

Furthermore preferred examples of the glycine derivative are those having the formula (I) wherein $R^1$ is chlorophenyl group, either $R^2$ or $R^3$ is methyl group and the other is hydrogen atom and $R^4$ is free carboxyl group.

The glycine derivative (I) of the present invention may exist in the form of two optically active isomers due to one asymmetric carbon atom , and the present invention includes these optically active isomers and a mixture thereof.

According to the present invention, the glycine derivatives (I) can be prepared, for instance, by the steps of:

(1.a) condensing a compound having the formula (II):

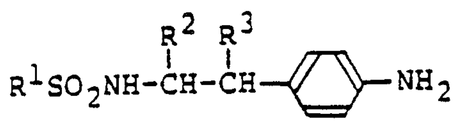
(II)

wherein $R^1$, $R^2$ and $R^3$ are the same as defined above, or a salt thereof with a compound of the formula (III):

$XCH_2(CONH\text{-}Q)_m R^{41}$ (III)

wherein X is a reactive residue, $R^{41}$ is carboxyl group or a protected carboxyl group, Q and m are the same as defined above, or a salt thereof, or

(1.b) condensing a compound having the formula (IV):

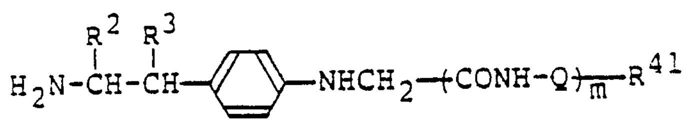
(IV)

wherein $R^2$, $R^3$, $R^{41}$, Q and m are the same as defined above, or a salt thereof with a sulfonic acid compound having the formula (V):

$R^1SO_3H$ (V)

wherein $R^1$ is the same as defined above or a reactive derivative thereof; and,

(2) if required, further removing the protecting group from the product when $R^{41}$ is the protected carboxyl group.

The glycine derivative having the formula (I) wherein m is 1 can also be prepared by condencing a compound of the formula (I-a):

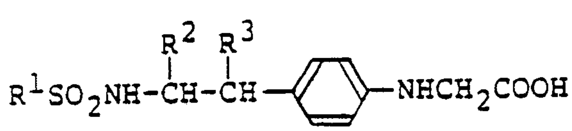
(I-a)

wherein $R^1$, $R^2$ and $R^3$ are the same as defined above, or a reactive derivative at carboxyl group thereof with an amine compound of the formula (VI):

$H_2N\text{-}Q\text{-}R^{41}$ (VI)

wherein $R^{41}$ and Q are the same as defined above or a salt thereof, and if required, further removing the protecting group therefrom when $R^{41}$ is the protected carboxyl group.

Any protecting groups which can be removed by conventional manner such as hydrolysis, reduction, solvolysis or acid-treatment can be used as the protecting group at carboxyl group of the starting compounds (III), (IV) and (VI). Examples of such a protecting group include a lower alkyl group, a substituted or unsubstituted phenyl-lower alkyl group (e.g., benzyl group, p-methoxybenzyl group or p-nitrobenzyl group), benzhydryl group, and the like. On the other hand, preferable examples of the reactive

residue X include a halogen atom, a lower-alkylsulfonyloxy group, a substituted or unsubstituted phenylsulfonyloxy group (e.g., benzenesulfonyloxy group, p-toluenesulfonyloxy group), and the like. Further, as the reactive derivatives of the compound (V) corresponding sulfonyl halides thereto can be preferably used.

The condensation reaction [step 1.a] of the compound (II) and the compound (III) and the condensation reaction [step 1.b] of the compound (IV) and the compound (V) or a reactive derivative thereof can be carried out by means of a usual method. The condensation reaction can be carried out in the precence or absence of a condensing agent, preferably carried out in the presence of the condensing agent. As the condensing agent, a usual condensing agent such as a dehydrating agent (e.g., carbonyldiimidazole or dicyclohexylcarbodiimide) or an acid acceptor (e.g., an alkali metal carbonate, an alkali metal hydroxide, an alkali metal hydrogencarbonate, pyridine, an organic amine such as a tertiary amine which has three lower alkyl groups) can be suitably used. The condensation reaction can be carried out in an appropriate solvent (e.g., acetone, chloroform, alkanol, methylene chloride, tetrahydrofuran, dimethyl sulfoxide, dimethylformamide, hexamethylphosphoric triamide) at a temperature of -50° to 50° C.

The condensation reaction of the compound (I-a) or the reactive derivative thereof at carboxyl group and an amine compound (VI) can be carried out by a usual method in the presence or absence of a condensing agent. As the reactive derivative at carboxyl group of the compound (I-a), for example, a corresponding acid halide or active ester thereto, and the like can be suitably used. The condensing agent as mentioned above can be used in the condensation reaction of the compound (I-a) or its derivataive with the amine compound (VI). These condensation reactions can be carried out in an appropriate solvent (e.g., tetrahydrofuran, methylene chloride) at a temperature of -50° to 50° C.

In carrying out the above reactions, the compounds (II), (III), (IV), (I-a) and (VI) may be used in the form of salts thereof such as alkali metal salts, alkaline earth metal salts and the like. The compounds (II), (IV) and (VI) may be also used in the form of salts thereof such as mineral acid salts.

When $R^{41}$ is the protected carboxyl group in thus obtained product, the protecting group may be removed from the product in a known method such as hydrolysis, reduction, solvolysis or acid-treatment, as occasion demands.

All of the above reactions proceed without racemization, and hence, the glycine derivative (I) of the present invention can be obtained in an optically active form by the use of an optically active compound (I-a), (II) or (IV) as a starting compound.

As mentioned hereinbefore, the glycine derivative (I) of the present invention and the salt thereof show the potent $TxA_2$ antagonistic activities and are suitable for use as platelet aggregation-inhibitors. For instance, they are useful for the treatment, amelioration and/or prophylaxis of a variety of thromboses or embolisms such as cerebral thrombosis, coronary artery thrombosis, pulmonary thrombosis, pulmonary embolism, peripheral vascular embolism and thromboangiitis.

Therefore a further object of the present invention is to provide a pharmaceutical.composition which comprises a glycine derivative or a salt thereof according to the invention and if necessary a pharmaceutical acceptable carrier thereof.

A further objet of the invention is to provide a pharmaceutical composition having a platelet aggregation inhibitory activity which comprises a glycine derivative or a salt thereof according to the invention and if necessary a pharmaceutical acceptable carrier thereof.

A further objet of the invention is to provide a pharmaceutical composition having a $TxA_2$ antagonistic activity, which comprises a glycine derivative or a salt thereof according to the invention and if necessary a pharmaceutical acceptable carrier thereof.

The glycine derivative (I) of the present invention can be used for pharmaceutical use either in the form of a free base or a salt thereof. For pharmaceutical use, the salts of the glycine derivative (I) are preferably pharmaceutically acceptable salts, for example, inorganic or organic salts such as alkali metal salts (e.g., sodium salt or potassium salt), alkaline earth metal salts (e.g., calcium salt, magnesium salts), heavy metal salts (e.g., zinc salt), ammonium salts, organic amine salts (e.g., triethylamine salt, ethanolamine salt), pyridine salts, basic amino acid salts (e.g., lysine salt, arginine salt or histidine salt), and the like.

The glycine derivative (I) or the salt thereof may be administered either orally or parenterally to a warm-blooded animal, including human beings, and may also be used in the form of a pharmaceutical preparation containing the same compound in admixture with pharmaceutical excipients suitable for oral or parenteral administration. The pharmaceutical preparations may be in solid forms such as tablets, capsules and powders, or in liquid forms such as solutions, suspensions and emulsions. Moreover, when administered parenterally, it may be used in the form of injections.

The compound (I) and a salt thereof may be administered either orally or parenterally and may also be used in the form of a pharmaceutical preparation containing the same compound in admixture with pharmaceutical excipients suitable for oral or parenteral administration. The pharmaceutical preparations

may be in solid form such as tablets, capsules or suppositories or in liquid form such as solutions, suspensions or emulsions. Moreover, when administered parenterally, the pharmaceutical preparation may be used in the form of injections.

The glycine derivative (I) of the present invention did not show any toxicity when they were orally administered in a dose of 1,000 mg/kg to mice. These results reveal that the glycine derivative (I) of the present invention has a low toxicity.

The glycine derivative (I) of the present invention or a pharmaceutically acceptable salt thereof is administered in such a dose that the desired activity is achieved without any side-effect. Though the actual dose varies according to a medication, an age of patient, a weight of patient, a condition of patient and a disease to be treated, a dosage of the glycine derivative (I) of the present invention per day for adults is usually from about 0.01 to about 50 mg/kg, preferably about 0.05 to about 20 mg/kg.

Besides the starting compound (II) can be prepared, for instance, by condensing a compound of the formula (VII):

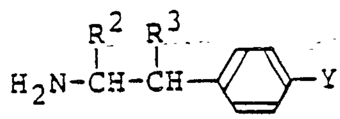

(VII)

wherein Y is nitro group or amino group, $R^2$ and $R^3$ are the same as defined above with the compound (V) or the reactive derivative thereof to give a compound (VIII):

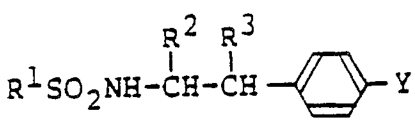

(VIII)

wherein Y, $R^1$, $R^2$ and $R^3$ are the same as defined above, and, when Y is nitro group, further converting Y into amino group by reduction.

Also, the starting compound (IV) can be prepared by condensing a compound (VII-a):

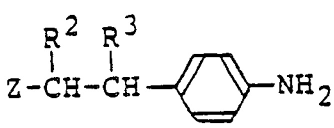

(VII-a)

wherein Z is amino group or a protected amino group, $R^2$ and $R^3$ are the same as defined above with the compound (III) and, when Z is the protected amino group, further removing the protecting group from the product.

The present invention is more particularly described and explained by means of the following Examples. It is to be understood, however, that the present invention is not limited to the Examples and various changes and modifications can be made without departing from the spirit and scope of the present invention.

[Inhibiting effect on arachidonic acid-induced pulmonary embolism (in vivo)]

An aqueous carboxymethylcellulose (hereinafter referred to as "CMC") solution containing a test compound (20 ml/kg) was orally administered to ddY-male mice (8 mice per group) fasted overnight. Three hours later, arachidonic acid (125 mg/10ml/kg) was injected to the tail vein of the mice to induce pulmonary embolism, and the recovery time (minute) of locomotive activity of the mice was compared with that of a control group of mice to which a 0.25 % aqueous CMC solution was administered instead of the test compound solution. The inhibiting effect of each test compound on arachidonic acid-induced pulmonary embolism was estimated in terms of a dose (minimum effective dose) required to induce at least 15 % decrease in the recovery time as compared with the control group. The results are shown in Table 1.

Table 1

| Test compound No.* | Minimum effective dose (mg/kg) |
|---|---|
| (Compound of the present invention) | |
| 1 | 1 |
| 2 | 1 |
| 3 | 1 |
| 4 | 3 |
| Known compound | 10 |

* Chemical name of test compounds are as follows:

| Test compound No. | Chemical name |
|---|---|
| 1 | Sodium N-{4-[2-[(4-chlorophenyl)sulfonylamino]-1-methylethyl]phenyl}glycinate |
| 2 | Sodium N-{4-[2-[(4-chlorophenyl)sulfonylamino]propyl]phenyl}glycinate |
| 3 | Sodium 3-{[[4-[2-(4-chlorophenyl)sulfonylamino-1-methylethyl]phenyl]amino]acetylamino}-n-propionate |
| 4 | Sodium 4-{[[4-[2-(4-chlorophenyl)sulfonylamino-1-methylethyl]phenyl]amino]acetylamino}-n-butyrate |

Known compound: Sodium N-[4-(2-benzenesulfonylaminoethyl)phenyl]glycinate

Example 1

To a mixture of 2.5 g of 1-(4-aminophenyl)-2-benzenesulfonylamino-1-methylethane, 1.09 g of sodium hydrogencarbonate and 15 mℓ of hexamethylphosphoric triamide was added dropwise 1.51 g of ethyl

bromoacetate under ice-cooling, and the mixture was stirred for 1 hour under ice-cooling, further for 30 minutes at room temperature. To the reaction mixture was added water, and then the resulting mixture was extracted with ethyl acetate. The extract was dried and evaporated to remove the solvent. The residue was purified by silica gel column chromatography (eluent; chloroform : ethanol = 20 : 1) and recrystallized from a mixture of ethyl acetate and n-hexane to give 1.89 g of ethyl N-[4-(2-benzenesulfonylamino-1-methylethyl)phenyl]glycinate.
Yield: 58 %

m.p. : 70°-71°C (m.p.: Melting point)

IR $\nu_{max}^{nujol}$ ($cm^{-1}$) 1740

Examples 2 to 12

The corresponding starting compounds were treated in the same manner as described in Example 1 to give each compound shown in Tables 2 and 3.

Table 2

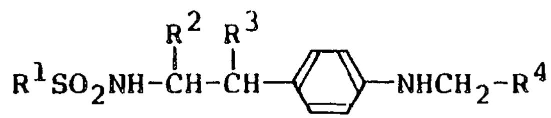

| Ex. No. | $R^1$ | $R^2$ | $R^3$ | $R^4$ | Physical properties |
|---|---|---|---|---|---|
| 2 | 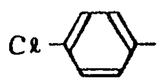 | H | $CH_3$ | $-COOCH_2CH_3$ | m.p.: 96°-98°C<br>IR*1: 1730 |
| 3 | 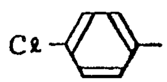 | $CH_3$ | H | $-COOCH_3$ | m.p.: 124°-127°C<br>IR*1: 1740 |
| 4 | 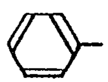 | $CH_3$ | H | $-COOCH_3$ | m.p.: 87°-89°C<br>IR*1: 1740 |

(Note)

IR*1 : IR $\nu_{max}^{nujol}$ ($cm^{-1}$)

Table 3

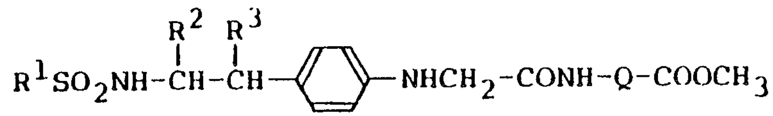

| Ex. No. | $R^1$ | $R^2$ | $R^3$ | Q | Physical property |
|---|---|---|---|---|---|
| 5 | 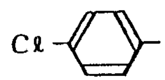 | H | $CH_3$ | $-(CH_2)_2-$ | $IR^{*2}$: 3380, 1730, 1660 |
| 6 | 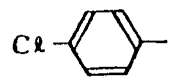 | H | $CH_3$ | $-(CH_2)_3-$ | $IR^{*2}$: 3380, 1730, 1650 |
| 7 | 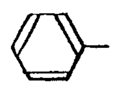 | H | $CH_3$ | $-(CH_2)_2-$ | $IR^{*2}$: 3280, 1730, 1660 |
| 8 | 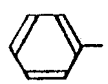 | H | $CH_3$ | $-(CH_2)_3-$ | $IR^{*2}$: 3370, 1730, 1650 |
| 9 | 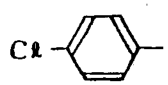 | $CH_3$ | H | $-(CH_2)_2-$ | $IR^{*3}$: 1730, 1670 |

- continued -

- continued -

| Ex. No. | $R^1$ | $R^2$ | $R^3$ | Q | Physical property |
|---|---|---|---|---|---|
| 10 | 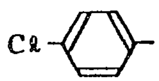 | $CH_3$ | H | $-(CH_2)_3-$ | $IR^{*3}$: 1730, 1670 |
| 11 | 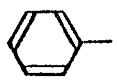 | $CH_3$ | H | $-(CH_2)_2-$ | $IR^{*3}$: 1730, 1670 |
| 12 | 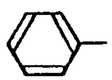 | $CH_3$ | H | $-(CH_2)_3-$ | $IR^{*3}$: 1730, 1665 |

(Notes)

$IR^{*2}$ : $IR\ \nu_{max}^{liquid}$ $(cm^{-1})$

$IR^{*3}$ : $IR\ \nu_{max}^{CHCl_3}$ $(cm^{-1})$

## Example 13

To a solution of 1.8 g of ethyl N-{4-[2-[(4-chlorophenyl)sulfonylamino]-1methylethyl]phenyl}glycinate in 30 mℓ of ethanol was added 5.3 mℓ of a 1 N aqueous solution of sodium hydroxide, and the mixture was stirred for 17 hours at room temperature. After the reaction was completed, the solvent was distilled away, and then the residue was recrystallized from an aqueous isopropyl alcohol solution to give 1.11 g of sodium N-{4-[2-[(4-chlorophenyl) sulfonylamino]-1-methylethyl]phenyl}glycinate as colorless crystals.
Yield : 63 %
m.p. : 242°-244° C (decomp.)

$$IR\ \nu_{max}^{nujol}\ (cm^{-1})\ \ 3380,\ 3100,\ 1600$$

$$\text{free carboxylic acid: } IR\ \nu_{max}^{nujol}\ (cm^{-1})\ \ 1720$$

## Examples 14 to 16

The corresponding starting compounds were treated in the same manner as described in Example 13 to give each compound shown in Table 4.

Table 4

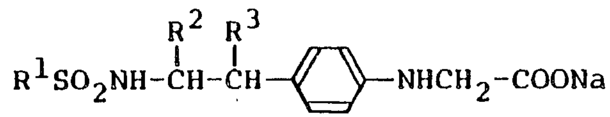

| Ex.No. | $R^1$ | $R^2$ | $R^3$ | Physical properties |
|---|---|---|---|---|
| 14 | 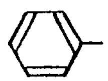 | H | $CH_3$ | IR*4: 1610, 1590<br>free carboxylic acid:<br>m.p.:123°-125°C (decomp.) |
| 15 | 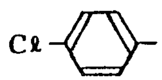 | $CH_3$ | H | m.p. 215-217°C<br>IR*1: 1600<br>free carboxylic acid:<br>m.p.:124°-126°C (decomp.) |
| 16 | 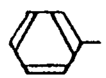 | $CH_3$ | H | IR*4:1610<br>free carboxylic acid:<br>m.p.:126°-128°C (decomp.) |

(Notes)

IR*1 : IR $\nu_{max}^{nujol}$ ($cm^{-1}$)

IR*4 : IR $\nu_{max}^{KBr}$ ($cm^{-1}$)

## Example 17

In a dried mixed solvent of 30 mℓ of methylene chloride and 30 mℓ of tetrahydrofuran was dissolved 1.96 g of N-{4-[2-(4-chlorophenyl)-sulfonylamino-1-methylethyl]phenyl}glycine. To the mixture was added 0.83 g of carbonyldiimidazole under ice-cooling, and the whole was stirred for 1.5 hours at room temperature. Successively, 714 mg of methyl 3-aminopropionate hydrochloride and 520 mg of triethylamine were added thereto. After the mixture was reacted for 1 hour at room temperature, 1 mℓ of methanol was added thereto, and the solvent was distilled away. The residue was extracted with ethyl acetate. After the extract was washed and dried, the solvent was distilled away. Further the residue was purified by silica gel column chromatography (eluent; chloroform : ethyl acetate = 4 : 1) to give 1.58 g of methyl 3-{[[4-[2-(4-chlorophenyl)sulfonylamino-1-methylethyl]phenyl]amino]acetylamino}-n-propionate.

The physical property of the compound thus obtained was the same as that of the compound obtained in Example 5.

Besides, the compounds described in Examples 6 to 12 can be obtained by treating free carboxylic acid compounds obtained in Examples 13 to 16 with the corresponding amino acids in the same manner as described above.

## Examples 18 to 25

The compounds obtained in Examples 5 to 12 were treated in the same manner as described in Example 13 to give the compounds shown in Table 5.

Table 5

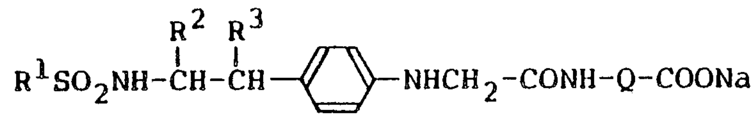

| Ex. No. | $R^1$ | $R^2$ | $R^3$ | Q | Physical properties |
|---|---|---|---|---|---|
| 18 | 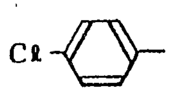 | H | $CH_3$ | $-(CH_2)_2-$ | IR*1: 1650<br>free carboxylic acid:<br>IR*1: 3500, 3260, 1710, 1660 |
| 19 | 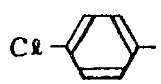 | H | $CH_3$ | $-(CH_2)_3-$ | IR*1: 1650<br>free carboxylic acid:<br>IR*1: 3500, 3260, 1710, 1660 |
| 20 | 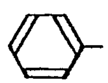 | H | $CH_3$ | $-(CH_2)_2-$ | IR*1: 1650 |
| 21 | 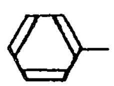 | H | $CH_3$ | $-(CH_2)_3-$ | IR*1: 1650<br>free carboxylic acid:<br>IR*1: 1720, 1650 |

- continued -

- continued -

| Ex. No. | $R^1$ | $R^2$ | $R^3$ | Q | Physical properties |
|---|---|---|---|---|---|
| 22 | 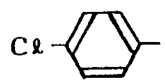 | $CH_3$ | H | $-(CH_2)_2-$ | IR*1: 1635<br>free carboxylic acid:<br>IR*1: 1715, 1665 |
| 23 | 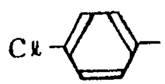 | $CH_3$ | H | $-(CH_2)_3-$ | IR*1: 1655<br>free carboxylic acid:<br>IR*1: 1705, 1665 |
| 24 | 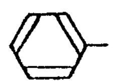 | $CH_3$ | H | $-(CH_2)_2-$ | IR*1: 1650<br>free carboxylic acid:<br>IR*1: 1720, 1660 |
| 25 | 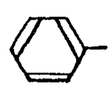 | $CH_3$ | H | $-(CH_2)_3-$ | IR*1: 1655<br>free carboxylic acid:<br>IR*1: 1710, 1660 |

(Note)

IR*1 : IR $\nu_{max}^{nujol}$ ($cm^{-1}$)

[Preparation of Starting Compounds]

## Comparative Example 1

(1) To a mixture of 3.48 g of sodium hydrogencarbonate, 20 mℓ of water and 30 mℓ of ethyl acetate was added 3.0 g of 2-amino-1-methyl-1-(4-nitrophenyl)ethane hydrochloride under ice-cooling and stirring, and then 2.69 g of benzenesulfonyl chloride was added thereto. The mixture was stirred for 20 minutes under ice-cooling, further for 40 minutes at room temperature. Then, ethyl acetate layer was separated, washed and successively dried. The solvent was distilled away. The residue was purified by silica gel column chromatography (eluent; chloroform : n-hexane : ethyl acetate = 7 : 2 : 1) and recrystallized from a mixture of chloroform and n-hexane to give 3.54 g of 2-benzenesulfonylamino-1-methyl-1-(4-nitrophenyl)-ethane.

$$IR\ \nu_{max}^{nujol}\ (cm^{-1})\quad 3300,\ 3280,\ 1590$$

(2) To a solution of 3.5 g of the product obtained in the step (1) in 150 mℓ of ethanol was added 0.35 g of 10 % palladium-carbon and the mixture was subjected to catalytic reduction. After the reduction was completed, the catalyst was filtered off and the solvent was distilled away. The residue was recrystallized from a mixture of chloroform and n-hexane to give 2.62 g of 1-(4-aminophenyl)-2-benzensulfonylamino-1-methylethane.
m.p. : 138°-141°C

$$IR\ \nu_{max}^{nujol}\ (cm^{-1})\quad 3440,\ 3360,\ 1620$$

## Comparative Example 2

(1) The corresponding starting compound was treated in the same manner as described in Comparative Example 1, the step (1) to give 1-(4-nitrophenyl)-2-(4- chlorophenyl)sulfonylamino-1-methylethane.
m.p. : 114°-116°C (recrystallization from a mixture of chloroform and n-hexane)

(2) To 20 mℓ of ethanol was added 3.1 g of the product obtained in the step (1). Successively, 9.86 g of tin(IV)chloride dihydrate was added over 5 minutes at 70°C with stirring. After the mixture was stirred for 3 hours at 70°C, the reaction mixture was poured onto 100 g of ice. Then, the mixture was made alkaline with a 10 % aqueous solution of sodium hydroxide and a precipitate was filtered off. The filtrate was extracted with methylene chloride and the extract was dried. Then, the solvent was distilled away. The residue was crystallized with a mixture of chloroform and n-hexane to give 2.06 g of 1-(4-aminophenyl)-2-(4-chlorophenyl)sulfonylamino-1-methylethane.
m.p. : 182°-183°C

## Comparative Example 3

To a suspension of 6.69 g of 2-amino-1-(4-aminophenyl)propane dihydrochloride in 50 mℓ of pyridine

and 30 mℓ of methylene chloride was added 18 mℓ of diazabicyclo[5.4.0]undec-7-ene at room temperature. Successively, a solution of 5.56 g of benzenesulfonyl chloride in 40 mℓ of methylene chloride was added dropwise thereto at -50° C and the mixture was stirred at -50° C for 2 hours. The temperature of reaction mixture was elevated to room temperature, and the solvent was distilled away. The residue was extracted with ethyl acetate. The extract was washed, then dried, the solvent was distilled away. The residue was purified by silica gel column chlomatography (eluent; chloroform : ethyl acetate = 4 : 1) and crystallized with diethyl ether to give 4.66 g of 1-(4-aminophenyl)-2-benzenesulfonylaminopropane as light yellow crystals.
m.p. : 89°-91° C

Comparative Example 4

The corresponding starting compound was treated in the same manner as described in Comparative Example 3 to give 1-(4-aminophenyl)-2-(4-chlorophenyl)-sulfonylaminopropane.
m.p. : 129°-130° C

## Claims

1. A glycine derivative having the formula (I):

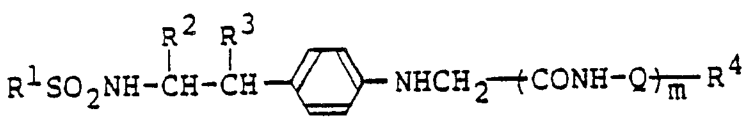

(I)

wherein $R^1$ is a substituted or unsubstituted phenyl group; either $R^2$ or $R^3$ is a lower alkyl group and the other is hydrogen atom; $R^4$ is a protected or nonprotected carboxyl group, Q is a lower alkylene-group and m is O or 1
or a salt thereof.

2. The glycine derivative of Claim 1, wherein said $R^1$ is phenyl group or a halogenophenyl group.

3. The glycine derivative of Claim 2, wherein said $R^4$ is free carboxyl group or a lower alkoxycarbonyl group.

4. The glycine derivative of Claim 1, wherein said $R^1$ is chlorophenyl group; either said $R^2$ or said $R^3$ is methyl group and the other is hydrogen atom; and said $R^4$ is free carboxyl group.

5. A process for preparing a glycine derivative having the formula (I):

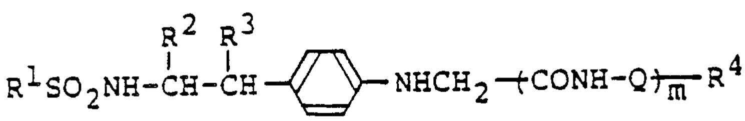

(I)

wherein $R^1$ is a substituted or unsubstituted phenyl group; either $R^2$ or $R^3$ is a lower alkyl group and the other is hydrogen atom, $R^4$ is a protected or nonprotected carboxyl group, Q is a lower alkylene group and m is O or 1
or a salt thereof, which comprises the step(s) of:

(1.a) condensing a compound having the formula (II):

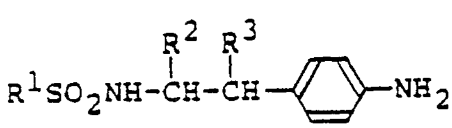

(II)

wherein $R^1$, $R^2$ and $R^3$ are the same as defined above or a salt thereof with a compound having the formula

(III):

$XCH_2\text{-}(CONH\text{-}Q)_m\text{-}R^{41}$ (III)

wherein X is a reactive residue, $R^{41}$ is carboxyl group or a protected carboxyl group, and Q and m are the same as defined above, or a salt thereof, or

(1.b) condensing a compound having the formula (IV):

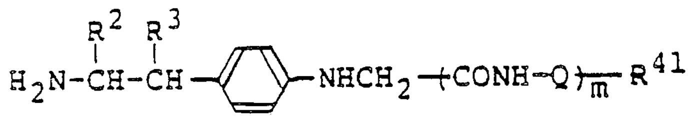

(IV)

wherein $R^2$, $R^3$, $R^{41}$, Q and m are the same as defined above, or a salt thereof with a sulfonic acid compound having the formula (V):

$R^1SO_3H$ (V)

wherein $R^1$ is the same as defined above, or a reactive derivative thereof, and,

(2) if required, further removing the protecting group from the product when $R^{41}$ is the protected carboxyl group.

6. A process for preparing a glycine derivative having the formula :

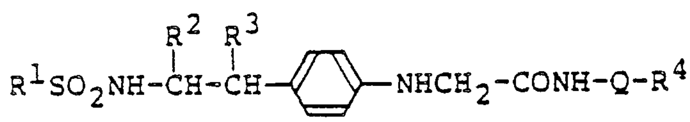

wherein $R^1$ is a substituted or unsubstituted phenyl group; either $R^2$ or $R^3$ is a lower alkyl group and the other is hydrogen atom, $R^4$ is a protected or nonprotected carboxyl group and Q is a lower alkylene group or a salt thereof, which comprises the step(s) of :

condensing a compound of the formula (I-a):

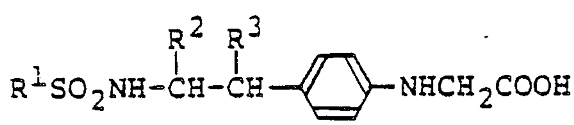

(I-a)

wherein $R^1$, $R^2$ and $R^3$ are the same as defined above, or a reactive derivative at carboxyl group thereof with an amine compound having the formula (VI):

$NH_2\text{-}Q\text{-}R^{41}$ (VI)

wherein Q is the same as defined above or a salt thereof and $R^{41}$ is carboxyl group or a protected carboxyl group, or a salt thereof, and if required, further removing the protecting group therefrom when $R^{41}$ is the protected carboxyl group.

7. A pharmaceutical composition which comprises:

a glycine derivative having the formula (I):

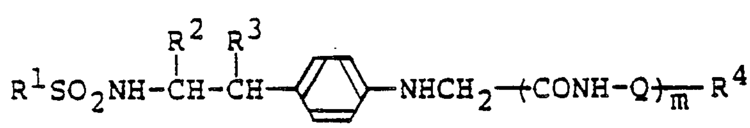

(I)

wherein $R^1$ is a substituted or unsubstituted phenyl group; either $R^2$ or $R^3$ is a lower alkyl group and the other is hydrogen atom; $R^4$ is a protected or nonprotected carboxyl group, Q is a lower alkylene group and m is O or 1

or a pharmaceutically acceptable salt thereof and if necessary a pharmaceutically acceptable carrier thereof.

8. The composition of Claim 7, wherein said $R^1$ is phenyl group or a halogenophenyl group.

9. The composition of Claim 8, wherein said $R^4$ is free carboxyl group or a lower alkoxycarbonyl group.

10. The composition of Claim 7, wherein said $R^1$ is chlorophenyl group; either said $R^2$ or said $R^3$ is methyl group and the other is hydrogen atom; and said $R^4$ is free carboxyl group.

11. A pharmaceutical composition effective in the therapeutic or prophylactic treatment of thrombosis or embolisms wich comprises a glycine derivative according to claims 1 to 4 or a pharmaceutically acceptable salt thereof.

12. A pharmaceutical composition having a platelet aggregation inhibitory activity which comprises a glycine derivative according to claims 1 to 4 or a pharmaceutically acceptable salt thereof.

13. A pharmaceutical composition having a $TxA_2$ antagonistic activity, which comprises a glycine derivative according to claims 1 to 4 or a pharmaceutically acceptable salt thereof.

European Patent Office

# EUROPEAN SEARCH REPORT

Application Number

EP 89 40 3605

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | DE-A-3 622 865 (BAYER AG)<br>* the whole document *<br>--- | 1-6,8-13 | C 07 C 311/19<br>C 07 C 311/16<br>A 61 K 31/195 |
| A | EP-A-0 255 728 (TANABE SEIYAKU CO. LTD.)<br>* pages 14-18; claims *<br>----- | 1-6,8-13 | |

| TECHNICAL FIELDS SEARCHED (Int. Cl.5) |
|---|
| C 07 C 311/00<br>A 61 K 31/00 |

The present search report has been drawn up for all claims

| Place of search | Date of completion of the search | Examiner |
|---|---|---|
| BERLIN | 03-02-1990 | RUFET J.M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document